# FASCICULE DE BREVET EUROPEEN

(11) **EP 0 608 207 B1**
(45) Date de publication et mention de la délivrance du brevet: **14.10.1998**
(21) Numéro de dépôt: 94870001.8
(22) Date de dépôt: 05.01.1994
(51) Int. Cl.: A61K 9/51, B01J 13/18

(54) **Compositions pharmaceutiques contenant des nanocapsules**
Arzneizusammensetzungen enthaltend Nanokapseln
Nanocapsule containing pharmaceutical compositions

(30) Priorité: 18.01.1993 GB 9300875
(43) Date de publication de la demande: 27.07.1994
(73) Titulaire: U C B, S.A., 1070 Bruxelles (BE)
(72) Inventeur: Vranckx, Henri, B-1190 Bruxelles (BE); Demoustier, Martine, B-1080 Bruxelles (BE); Deleers, Michel, B-1630 Linkebeek (BE)
(74) Mandataire: Debled, Thierry

(56) Documents cités:
- EP-A- 0 447 318
- DE-A- 3 341 001
- FR-A- 2 515 960
- DATABASE WPI Week 8520, Derwent Publications Ltd., London, GB; AN 85-120200 (20) & JP-A-60 061 521 (KYOWA HAKKO KOGYO) 9 Avril 1985
- CHEMICAL ABSTRACTS, vol. 118, no. 24, 14 Juin 1993, Columbus, Ohio, US; abstract no. 240600u, & PROC. PROGRAM INT. SYMP. CONTROLL. RELEASE BIOACT. MATER.,18TH,97-8.EDITED BY:KELLAWAY,IAN W. CONTROLLED RELEASE SOC.:DEERFIELD, ILL. 1991 C.MICHEL ET AL. 'Isobutyl cyanoacrylate nanoparticles as a drug carrier for oral administration of insulin'
- Diabetes 1988, 37, 246-251 (Damge et al.)"New Approach for Oral Administration of Insulin With Polyalkylcyanoacrylate Nanocapsules as Drug Carrier"

## Description

La présente invention se rapporte à de nouvelles compositions pharmaceutiques, et plus particulièrement, à des compositions pharmaceutiques pour l'administration par voie orale se présentant sous forme d'une suspension colloïdale de nanocapsules de poly(2-cyanoacrylate d'alkyle) dans une phase huileuse et qui renferment une solution ou une suspension aqueuse d'une substance thérapeutiquement active. Elle concerne également le procédé de préparation de ces compositions.

On connaît déjà des compositions pharmaceutiques sous forme de capsules renfermant une phase aqueuse qui contient un médicament.

Ainsi, dans la demande de brevet allemand 3341001, on décrit des nanoparticules et des nanocapsules d'un diamètre moyen compris entre 200 et 900 nanomètres, qui renferment une phase interne aqueuse ou hydrophile contenant au moins 3% d'un médicament ou d'une autre substance biologiquement active. La paroi des nanocapsules est formée par polymérisation d'un monomère biodégradable tel qu'un 2-cyanoacrylate d'alkyle. Pour la formation des nanocapsules, le monomère est ajouté en solution dans le chloroforme anhydre à une émulsion préalablement préparée et constituée d'une phase interne hydrophile, de l'eau ou du méthanol alcalinisé par exemple, contenant le médicament en solution, et d'une phase externe hydrophobe constituée par un solvant organique non miscible à l'eau, le chloroforme, le toluène ou l'isooctane par exemple, contenant un agent tensioactif. A l'exemple 1 de cette demande de brevet, la phase interne est une solution aqueuse de bleu de méthylène, la phase externe organique est un mélange de chloroforme et de toluène et le monomère biodégradable est le 2-cyanoacrylate de méthyle. Après polymérisation, on sépare les nanocapsules de la phase externe ; à cet effet, les nanocapsules obtenues sont centrifugées, puis elles sont abondamment lavées et lyophilisées afin d'être débarrassées de tous les solvants organiques indésirables. On obtient une poudre qui doit finalement être reconditionnée pour donner une composition administrable en vue de l'usage médical.

Dans la demande de brevet japonais 61521/85, on décrit des microcapsules dont le diamètre est compris entre 1 et 500 micromètres et qui renferment une phase aqueuse contenant un médicament en solution ou en suspension dans une solution aqueuse d'un polymère biocompatible tel que l'albumine, le dextran, la gélatine ou le collagène. Ces microcapsules sont préparées par addition sous agitation d'un 2-cyanoacrylate d'alkyle dans lequel le radical alkyle renferme de 1 à 8 atomes de carbone et qui n'est pas dissous dans un solvant, à une émulsion du type eau dans l'huile, préparée par addition de la phase aqueuse à un solvant organique peu soluble dans l'eau, qui peut éventuellement contenir un agent tensioactif. Après polymérisation du 2-cyanoacrylate d'alkyle à l'interface des deux phases, on isole les microcapsules. A cet effet, la suspension des microcapsules dans le solvant organique est diluée par addition de solvants hydrocarbonés à bas point d'ébullition tel que l'éther de pétrole ou l'hexane, et la suspension est ensuite filtrée sur un filtre membrane. Le résidu de filtration est abondamment lavé avec les mêmes solvants et ensuite séché sous vide à 40°C pour éliminer toutes traces de solvants organiques et d'eau. La préparation pharmaceutique ainsi obtenue se présente sous la forme de microcapsules qui renferment le médicament dissous ou dispersé dans la solution aqueuse du polymère biocompatible. Cette préparation est destinée à être administrée par injection et doit être reconditionnée en vue de l'usage médical. Dans les exemples de cette demande de brevet, plus particulièrement, le solvant organique est une huile de graine de coton, le polymère biocompatible est l'albumine de sérum de bovin et le médicament est un agent carcinostatique. Cette demande de brevet japonais ne révèle que des solutions injectables, mais ne fait aucune mention de compositions pharmaceutiques qui conviennent pour une administration par voie orale.

Les compositions pharmaceutiques préparées selon les procédés décrits ci-dessus présentent plusieurs inconvénients importants.

En premier lieu, il est bien connu que pour obtenir une bonne absorption des substances thérapeutiquement actives dans le tractus gastro-intestinal, il y a tout intérêt à ce que le diamètre des particules encapsulant ces substances soit le plus faible possible. C'est la raison pour laquelle des particules qui ont un diamètre inférieur à 1 micromètre conviennent beaucoup mieux pour préparer une composition pharmaceutique destinée à être administrée par voie orale. Or, par le procédé selon la demande de brevet japonais, on obtient exclusivement des microcapsules dont le diamètre est supérieur à 1 micromètre et généralement de 1 à 500 micromètres.

En second lieu, les solvants organiques (isooctane, méthanol alcalinisé, chloroforme, toluène, éther de pétrole) qui sont généralement nécessaires pour préparer les capsules sont des solvants inacceptables d'un point de vue pharmaceutique à cause de leur toxicité élevée ; ils ne permettent donc pas l'administration directe de la préparation. Ceci entraîne dès lors la mise en oeuvre d'une étape supplémentaire délicate qui comprend la séparation des capsules à l'état pur, par exemple par filtration sur membrane ou par ultracentrifugation, suivie d'une purification poussée afin de débarrasser complètement les capsules de toutes traces de solvants incompatibles avec une application thérapeutique. Il en résulte qu'après purification, les capsules nécessitent une reformulation adéquate pour être finalement administrables sans inconvénient.

En troisième lieu, dans certains cas, comme celui de la demande de brevet japonais précitée, la préparation des capsules implique obligatoirement la présence d'une macromolécule, par exemple l'albumine de sérum de bovin ou du dextran. Il faut noter toutefois que ces macromolécules d'origine animale peuvent provoquer des réactions immunologiques indésirables et les chocs anaphylactiques aux dextrans sont spectaculaires et bien connus.

Etant donné que la voie orale reste une des voies les plus prisées pour l'administration de médicaments, les injections étant en général considérées comme plus douloureuses par les patients et redoutées par ceux-ci, et que les capsules submicroscopiques contenant des substances pharmaceutiques actives ont donné des résultats encourageants lors de l'administration de celles-ci en tant que vecteurs de médicaments, notamment par voie parentérale, il serait particulièrement intéressant de pouvoir disposer de compositions pharmaceutiques pour l'administration par voie orale sous forme de capsules submicroscopiques renfermant la substance à activité thérapeutique en solution ou en suspension dans une phase aqueuse, mais qui ne présentent pas les inconvénients des compositions pharmaceutiques connues de l'état de la technique.

En effet, cette forme d'administration sous forme de capsules submicroscopiques est nécessaire, principalement dans le cas de médicaments qui peuvent être dégradés dans l'estomac, tels que les médicaments à structure peptidique.

En particulier, il serait très souhaitable que les capsules submicroscopiques soient formées d'un matériau biodégradable, que leur taille soit suffisamment faible pour laisser présager d'une bonne absorption dans le tractus gastro-intestinal et qu'en même temps, elles renferment sous une forme encapsulée, une quantité élevée de la substance pharmaceutiquement active. De plus, si ces compositions pouvaient être directement administrables sans nécessiter des étapes supplémentaires d'isolement, de purification et de reconditionnement, cela constituerait une simplification technique importante et un avantage non négligeable.

La demanderesse vient présentement de découvrir de nouvelles compositions pharmaceutiques sous forme de nanocapsules qui répondent pleinement à ces objectifs.

La présente invention a donc pour objet une composition pharmaceutique sous forme d'une suspension colloïdale de nanocapsules, qui est caractérisée en ce qu'elle comprend une phase huileuse constituée essentiellement par une huile contenant en solution un agent tensioactif et en suspension, des nanocapsules d'un diamètre inférieur à 500 nanomètres, renfermant une phase aqueuse constituée essentiellement par une solution ou une suspension d'une substance à activité thérapeutique et d'un agent tensioactif dans l'eau, dont le pH est compris entre 1 et 7, la paroi des dites nanocapsules étant formée d'un poly(2-cyanoacrylate d'alkyle) dont le radical alkyle renferme de 1 à 6 atomes de carbone.

La présente invention a également pour objet un procédé de préparation de la composition pharmaceutique décrite ci-dessus, qui est caractérisé en ce que
a) on prépare une phase aqueuse constituée essentiellement par une solution ou une suspension d'une substance à activité thérapeutique et d'un agent tensioactif dans l'eau, dont le pH est compris entre 1 et 7 ;
b) on ajoute lentement, sous agitation, cette phase aqueuse dans une phase huileuse constituée essentiellement par une huile contenant en solution un agent tensioactif ;
c) on ajoute, sous agitation, à l'émulsion de type eau dans l'huile ainsi obtenue, au moins un 2-cyanoacrylate d'alkyle dont le radical alkyle renferme de 1 à 6 atomes de carbone, tel quel, sans solvant, puis on laisse la polymérisation s'effectuer à la température ambiante pendant une durée de temps suffisante pour que le 2-cyanoacrylate d'alkyle introduit soit polymérisé, et on recueille comme produit du procédé une suspension dans la phase huileuse de nanocapsules de diamètre inférieur à 500 nanomètres, renfermant ladite phase aqueuse.

La phase huileuse des compositions pharmaceutiques conformes à l'invention est constituée essentiellement par une huile neutre pharmaceutiquement acceptable contenant en solution un agent tensioactif. Cette huile peut être une huile végétale, une huile minérale ou tout composé huileux, insoluble dans l'eau, tel que le benzoate de benzyle ou les glycérides d'acides gras supérieurs en C₆ à C₁₈. Parmi ces derniers, on utilisera de préférence un Miglyol, comme par exemple le Miglyol 812, qui est une huile neutre formée d'un mélange de triglycérides d'acides gras saturés, à longueur de chaîne principalement de 8 à 10 atomes de carbone.

La phase aqueuse contenue dans la cavité centrale des nanocapsules présentes dans les compositions pharmaceutiques conformes à l'invention est constituée essentiellement par une solution ou une suspension d'une substance à activité thérapeutique, soluble ou insoluble dans l'eau ; elle contient également un agent tensioactif. Cette phase aqueuse contient de préférence également une certaine proportion d'alcool éthylique. La présence de cet alcool éthylique a pour effet de conférer aux parois des nanocapsules une rigidité plus grande. La quantité d'éthanol qui peut être ajoutée à la phase aqueuse est généralement comprise entre 100 µl et 1.000 µl par ml de phase aqueuse, et de préférence entre 200 µl et 500 µl par ml de phase aqueuse.

Selon l'invention, le pH de la phase aqueuse doit être ajusté entre 1 et 7 au moyen d'un acide pharmaceutiquement acceptable ou d'un tampon approprié, par exemple à 4,3 au moyen d'un tampon acétique. Si le pH dépasse une valeur de 7, on obtient des capsules d'un diamètre supérieur à 500 nanomètres, voire même des capsules de taille supérieure au micromètre.

Le rapport en volume de la phase aqueuse et de la phase huileuse est généralement compris entre 1/100 et 20/100, et de préférence entre 5/100 et 15/100.

Les agents tensioactifs qui peuvent être utilisés dans les compositions pharmaceutiques conformes à l'invention sont les agents tensioactifs pharmaceutiquement acceptables. Ils peuvent être des agents tensioactifs naturels, comme par exemple l'acide désoxycholique ou d'autres sels d'acides biliaires ou leurs dérivés, ou des agents tensioactifs synthétiques. Dans ce dernier cas, ils peuvent être soit de type anionique (comme le laurylsulfate de sodium ou le dioctylsulfosuccinate de sodium), soit de type cationique (comme les sels d'ammonium quaternaire), ou encore de type non ionique (comme les esters d'acides gras de sorbitanne polyoxyéthylénés ou non, par exemple, le Span 80, ou les dérivés mixtes de l'oxyde d'éthylène et de propylène glycol, par exemple, le Pluronic F68).

Des essais effectués par la demanderesse ont démontré la nécessité de la présence simultanée d'un agent tensioactif dans la phase aqueuse et dans la phase huileuse pour obtenir la taille des nanocapsules conformes à l'invention inférieure à 500 nanomètres, et de préférence plus grande que 80 nanomètres.

Les agents tensioactifs sont dissous respectivement dans le liquide approprié qui constitue la phase interne aqueuse ou la phase externe huileuse. Par exemple dans la phase aqueuse, on utilisera le laurylsulfate de sodium, le dioctylsulfosuccinate de sodium, les esters d'acide gras de sorbitanne polyoxyéthylénés et les dérivés mixtes d'oxyde d'éthylène et de propylène glycol et le polyéthoxyéthylène glycol, de préférence, on utilisera le laurylsulfate de sodium. Dans la phase huileuse, on utilisera des esters de sorbitanne et des sels solubles d'acides biliaires, de préférence, le mono-oléate de sorbitanne (Span 80) ou l'acide désoxycholique.

La concentration de l'agent tensioactif dans la phase aqueuse est généralement comprise entre 0,1% et 10%, de préférence entre 3% et 5% en poids d'agent tensioactif par volume de phase aqueuse.

La concentration de l'agent tensioactif dans la phase huileuse est généralement comprise entre 0,1% et 20%, de préférence entre 5% et 15% en poids d'agent tensioactif par volume de phase huileuse.

La paroi des nanocapsules se trouvant en suspension dans les compositions pharmaceutiques conformes à l'invention est formée d'un poly(2-cyanoacrylate d'alkyle) biodégradable obtenu par polymérisation micellaire d'au moins un 2-cyanoacrylate d'alkyle dans lequel le radical alkyle, qui peut être linéaire ou ramifié, contient 1 à 6 atomes de carbone. Les poly(2-cyanoacrylates d'alkyle) qui conviennent particulièrement bien pour la présente invention sont obtenus par polymérisation micellaire du 2-cyanoacrylate de n-butyle et du 2-cyanoacrylate de n-hexyle. Ces 2-cyanoacrylates d'alkyle peuvent être utilisés seuls ou en mélange, étant entendu que la paroi des nanocapsules peut consister en des polymères ou des copolymères de 2-cyanoacrylates d'alkyle.

Le 2-cyanoacrylate d'alkyle est ajouté tel quel, sans solvant, dans le milieu de polymérisation. Selon l'invention, cette polymérisation s'effectue à la température ambiante, pendant une durée de préférence comprise entre 3 et 8 heures.

La substance thérapeutiquement active utilisée dans les compositions pharmaceutiques conformes à l'invention est contenue dans la phase aqueuse présente dans la cavité centrale des nanocapsules. Toute substance médicamenteuse soluble dans l'eau, ou peu soluble dans l'eau, peut être utilisée dans ces compositions. En particulier, les nouvelles compositions conformes à l'invention conviennent avantageusement pour l'encapsulation de polypeptides comme la calcitonine, la somatostatine ou l'insuline et de polysaccharides comme l'héparine. Il est bien connu que ces produits sont très rapidement dégradés par les enzymes protéolytiques présentes dans le tractus gastro-intestinal, lorsqu'ils sont administrés par voie orale. Par contre, les compositions conformes à l'invention, appliquées à ces produits montrent une activité pharmaceutique significative lorsqu'elles sont administrées par cette même voie, grâce à l'encapsulation du principe actif.

La composition pharmaceutique conforme à l'invention et son procédé d'obtention qui viennent d'être décrits présentent des avantages très importants par rapport à l'état de la technique :
- obtention de nanocapsules d'un diamètre inférieur à 500 nanomètres, et plus particulièrement de 80 à 450 nanomètres, d'où une excellente absorption au niveau de la muqueuse intestinale ;
- stabilité physique excellente des nanocapsules obtenues après au moins 18 mois à la température ambiante et à 4°C ;
- étant donné l'absence complète de solvants organiques lors de la préparation des nanocapsules et de la composition pharmaceutique, la séparation et la purification laborieuse des nanocapsules afin de les débarrasser de toute trace de solvant incompatible avec une application thérapeutique ne se justifie plus ;
- étant donné l'absence de macromolécules d'origine animale telles que l'albumine et le dextran, il n'existe pas de risque de réactions immunologiques indésirables (choc anaphylactique par exemple) ;
- du fait que tous les constituants (huile, eau, agents tensioactifs) ont été choisis parmi les substances pharmaceutiquement acceptables, on obtient une composition pharmaceutique directement prête à l'emploi dont l'innocuité est assurée. La composition obtenue peut donc être administrée directement par la voie orale (ou éventuellement aussi par la voie intramusculaire) et ne nécessite pas l'isolement préalable des nanocapsules, leur purification et leur redispersion dans un milieu approprié. En outre, elle peut aussi être utilisée directement pour la mise en gélules pour l'administration orale ;
- la composition pharmaceutique est préparée par un procédé simple qui est facilement transposable à l'échelle industrielle.

Les exemples qui suivent sont donnés dans le but d'illustrer l'invention sans la limiter. Les exemples 1 à 5 et 8 sont plus particulièrement destinés à illustrer le procédé de préparation de la suspension dans l'huile des nanocapsules renfermant une phase aqueuse, les exemples 6, 7 et 9 à 16 illustrent une composition pharmaceutique sous forme de nanocapsules renfermant une substance à activité thérapeutique.

La stabilité physique de la préparation pharmaceutique ainsi obtenue est déterminée par l'observation directe de l'absence de formation de deux phases distinctes, aqueuse et huileuse, après plusieurs mois de repos, ainsi que par la mesure à intervalles de temps réguliers, de la taille moyenne des nanocapsules dispersées dans l'huile. La taille moyenne des nanocapsules est mesurée à l'aide d'un appareil "Coulter Model N4MD Sub-micron Particle Analyser".

### Exemple 1.

On ajoute lentement 1 ml de tampon acétique aqueux à pH 4,3 (composition du tampon : acide acétique glacial 2 g, acétate de sodium 2 g, chlorure de sodium 7,5 g, eau déminéralisée pour faire 1 litre) contenant 5% en poids par volume de laurylsulfate de sodium, à la température ambiante, sous vive agitation (1200 tpm) à 10 ml de Miglyol 812 (huile neutre formée d'un mélange de triglycérides d'acides gras saturés en C₈ à C₁₀) contenant 15% en poids par volume de mono-oléate de sorbitanne (Span 80). La suspension est agitée dans les mêmes conditions pendant 15 minutes, puis on ajoute 100 µl de 2-cyanoacrylate de n-butyle et on laisse la polymérisation du monomère s'effectuer pendant 240 minutes. On obtient ainsi en suspension dans l'huile, des nanocapsules à contenu aqueux ayant une taille moyenne de 255 nanomètres.

Ces nanocapsules ne présentent aucune variation significative de leur taille après conservation pendant au moins 18 mois à la température ordinaire et à 4°C.

### Exemple 2.

On prépare des nanocapsules en utilisant le même procédé qu'à l'exemple 1, mais on remplace le tampon acétique aqueux par un tampon phosphate aqueux à pH 7 ( composition du tampon : 39 ml d'une solution 0,2 molaire de NaH₂PO₄.H₂O, 61 ml d'une solution 0,2 molaire de Na₂HPO₄.2H₂O et 100 ml d'eau distillée).

Les nanocapsules à contenu aqueux obtenues ont une taille moyenne de 422 nanomètres.

### Exemple 3.

On opère dans les mêmes conditions qu'à l'exemple 1, mais en plus, la phase aqueuse contient en suspension 0,25 mg d'un colorant insoluble dans l'eau (FDC Blue 2 HT Aluminium, Lake cert. n° AA2041, Colorcon).

On obtient ainsi des nanocapsules renfermant une suspension aqueuse du colorant et ayant une taille moyenne de 246 nanomètres.

### Exemple 4.

On prépare des nanocapsules en utilisant le même procédé qu'à l'exemple 1, mais, cette fois, la phase huileuse est le Miglyol 829 (huile formée d'un mélange de triglycérides d'acides gras saturés en C₈ à C₁₀ et de 15 à 20% d'acide succinique).

On obtient ainsi des nanocapsules à contenu aqueux ayant une taille moyenne de 246 nanomètres.

### Exemple 5.

On prépare des nanocapsules selon le procédé de l'exemple 1, mais on remplace le 2-cyanoacrylate de n-butyle par le 2-cyanoacrylate de n-hexyle et on laisse polymériser pendant 8 heures.

On obtient des nanocapsules à contenu aqueux ayant une taille moyenne de 213 nanomètres.

La stabilité physique de ces nanocapsules est excellente : on n'observe aucune variation significative de leur taille après conservation à la température ordinaire pendant au moins 12 mois.

### Exemple 6.

On procède comme indiqué à l'exemple 1, mais la phase aqueuse contient aussi 1100 UI de calcitonine.

On obtient des nanocapsules ayant une taille moyenne de 177 nanomètres, dont la phase aqueuse renferme le médicament.

La stabilité physique de ces nanocapsules est excellente : il n'y a pas de variation significative de leur taille après au moins 12 mois de conservation à la température ambiante ou à 4°C.

### Exemple 7.

On prépare des nanocapsules selon le procédé de l'exemple 1, mais on remplace le 2-cyanoacrylate de n-butyle par le 2-cyanoacrylate de n-hexyle et la phase aqueuse contient en plus 1100 UI de calcitonine. Après 4 heures de polymérisation, on obtient des nanocapsules ayant une taille moyenne de 211 nanomètres, dont la phase aqueuse renferme le médicament. La stabilité physique de ces capsules est excellente, il n'y a pas de variation significative de leur taille pendant au moins 11 mois de conservation à la température ordinaire et à 4°C.

### Exemple 8.

On procède comme indiqué à l'exemple 1, mais la phase aqueuse contient 400 µl d'éthanol.

Après 4 heures de polymérisation, les nanocapsules à contenu aqueux obtenues ont une taille moyenne de 211 nanomètres.

La stabilité physique de ces nanocapsules est excellente : aucune variation significative de la taille n'apparaît après au moins 12 mois de conservation à la température ambiante ou à 4°C.

Ces nanocapsules qui renferment une faible quantité d'éthanol présentent une rigidité de la paroi plus élevée que celle des nanocapsules préparées à l'exemple 1 qui ne contiennent pas d'éthanol.

Cette rigidité de la paroi est déterminée au moyen d'un essai de centrifugation au cours duquel les nanocapsules en suspension dans l'huile sont soumises à une centrifugation à 20.000 tours par minute pendant deux heures. Les nanocapsules préparées dans cet exemple résistent parfaitement à cet essai.

### Exemple 9.

En utilisant le procédé de l'exemple 8, on prépare des nanocapsules dont la phase aqueuse est une solution à 0,025% (poids par volume) de bleu de méthylène dans l'eau. On obtient des nanocapsules ayant une taille moyenne de 205 nanomètres. Le pourcentage d'encapsulation du colorant atteint 98,3%.

### Exemple 10.

En utilisant le procédé de l'exemple 8, on prépare des nanocapsules dont la phase aqueuse contient 300 unités USP d'héparine. On obtient des nanocapsules ayant une taille moyenne de 137 nanomètres.

### Exemple 11.

En utilisant le procédé de l'exemple 8, on prépare des nanocapsules dont la phase aqueuse renferme 0,9 mg de somatostatine. On obtient des nanocapsules ayant une taille moyenne de 201 nanomètres.

### Exemple 12.

En utilisant le procédé de l'exemple 8, on prépare des nanocapsules dont la phase aqueuse contient 11.000 UI de calcitonine. La taille moyenne des nanocapsules en suspension dans cette préparation est de 211 nanomètres. On ajoute de l'acide désoxycholique dispersé dans du Miglyol 812 pour obtenir une concentration 0,02 molaire en acide désoxycholique dans le mélange.

### Exemple 13.

On procède comme indiqué à l'exemple 12, mais la phase aqueuse contient 110.000 UI de calcitonine. La taille moyenne des nanocapsules obtenues est de 225 nanomètres.

### Exemple 14.

Cet exemple a pour but de comparer l'efficacité de différentes préparations de calcitonine administrées à des rats par différentes voies : orale, duodénale, iléale et intraveineuse.

La réponse physiologique à l'administration de calcitonine dans l'organisme se manifeste par une diminution du taux de calcium circulant librement dans le sang.

La suspension de nanocapsules renfermant la calcitonine obtenue à l'exemple 13 est administrée à des rats Wistar mâles (poids : 200 ± 20 g), anesthésiés au pentobarbital (60 mg/kg), à la dose de 20 UI de calcitonine par animal, par la voie orale, duodénale ou iléale.

A titre comparatif, la même dose de calcitonine est administrée sous forme de solution aqueuse à des rats témoins par la voie intraveineuse.

Des échantillons de sang sont prélevés à intervalles réguliers dans une veine caudale. Après coagulation du sang et centrifugation, (2 x 10 minutes à 6.000 tpm), les sérums sont dilués et soumis à un dosage du calcium à l'aide d'un spectromètre d'absorption atomique. Les résultats des dosages correspondant à chaque mode d'administration permettent de dresser un graphique reprenant le pourcentage du taux sérique de calcium en fonction du temps. Les surfaces sous les courbes correspondant aux administrations par la voie orale, duodénale ou iléale sont calculées et comparées à celles obtenues par administration intraveineuse, qui correspond à l'activité maximale observée et qui, de ce fait, est posée égale à 100%.

Les résultats de ces tests montrent que 4 heures après que la préparation de calcitonine ait été administrée à l'animal, soit par voie orale, soit directement dans le duodénum ou dans l'iléon, on observe dans tous les cas une diminution du taux de calcium sérique correspondant à 73% de l'effet produit par l'administration intraveineuse de la même dose de calcitonine. L'activité obtenue après l'administration orale d'une composition pharmaceutique conforme à l'invention est donc aussi bonne que celle observée après administration de la même préparation par une voie plus délicate, la voie intraduodénale ou la voie iléale.

### Exemple 15.

On procède comme indiqué à l'exemple 1, mais le tampon acétique aqueux à pH 4,3 est remplacé par un tampon acétique aqueux à pH 3,6 (composition du tampon: 46,3 ml d'une solution 0,2 molaire d'acide acétique glacial, 3,7 ml d'une solution 0,2 molaire d'acétate de sodium et 50 ml d'eau distillée). En outre, la phase aqueuse contient 780 UI d'insuline-zinc de bovin et 200 µl d'éthanol.

On obtient des nanocapsules à contenu aqueux ayant une taille moyenne de 260 nanomètres.

### Exemple 16. Influence de l'éthanol sur la rigidité des nanocapsules.

Les observations de l'exemple 8, en ce qui concerne la rigidité des parois des nanocapsules en présence ou en l'absence d'éthanol dans la phase aqueuse trouvent une confirmation par le test effectué dans cet exemple.

Selon les conditions du test décrit à l'exemple 14, on administre à des rats, par la voie orale, la composition préparée à l'exemple 12 qui contient 400 µl d'éthanol et 0,02 mole d'acide désoxycholique ainsi qu'une composition préparée selon le même procédé de l'exemple 12, mais qui ne contient pas d'éthanol. Les résultats obtenus sont comparés, comme à l'exemple 14, avec l'effet obtenu par l'administration intraveineuse d'une dose identique de calcitonine en solution aqueuse.

Les résultats obtenus sont donnés au tableau I. La colonne 3 de ce tableau indique la diminution en % du taux de calcium sérique en quatre heures.

**TABLEAU I**

| Voie d'administration | Quantité d'éthanol (µl) | Taux de calcium (diminution en %) |
|---|---|---|
| orale | - | 7 |
| orale | 400 | 48 |
| intraveineuse | - | 100 |

Ce tableau montre que la composition qui ne contient pas d'éthanol provoque une diminution du taux de calcium sérique de 7% seulement en quatre heures, contrairement à une composition qui contient de l'éthanol qui, elle, provoque une diminution de 48%.

Cette différence s'explique par le fait que la rigidité des parois des nanocapsules qui ne contiennent pas d'éthanol est plus faible. Dès lors, une proportion importante de ces nanocapsules ne résiste pas au passage à travers l'estomac où une quantité non négligeable de calcitonine est digérée sous l'action des enzymes protéolytiques. Il en résulte qu'une plus faible quantité de calcitonine seulement atteint l'intestin où elle est effectivement résorbée, induisant ainsi une réponse physiologique de moindre intensité.

### Exemple 17. (comparatif).

En utilisant le procédé de l'exemple 8, on a tenté de préparer des nanocapsules dont la phase aqueuse contient une quantité croissante d'albumine de sérum bovin (BSA n° A-8022 commercialisé par Sigma Corp.) égale à 50, 100, 150 et 300 mg par millilitre de phase aqueuse.

En utilisant le procédé de l'exemple 12, on a également tenté de préparer des nanocapsules dont la phase aqueuse renferme aussi du BSA, à raison de 300 mg/ml.

Dans tous les cas, on obtient une préparation qui contient deux populations de capsules qui se distinguent par leurs tailles moyennes.

Dans le tableau II, on donne la taille moyenne des capsules et le pourcentage de chacune des deux populations de capsules obtenues en fonction de la teneur en BSA dans la phase aqueuse.

**TABLEAU II**

| BSA (mg/ml) | Calcitonine (UI) | Taille moyenne des capsules (nm) | Pourcentage |
|---|---|---|---|
| 50 | - | 294 | 75 |
| | | 3000 | 25 |
| 100 | - | 215 | 30 |
| | | 1040 | 70 |
| 150 | - | 520 | 22 |
| | | 1930 | 78 |
| 300 | - | 225 | 2 |
| | | 2610 | 98 |
| 300 | 1100 | 225 | 1 |
| | | 4690 | 99 |

Les résultats du tableau II montrent que la présence dans la phase aqueuse d'un biopolymère tel que l'albumine de sérum de bovin (comme dans la demande de brevet japonais 61521/85), ne permet pas d'obtenir une composition conforme à l'invention contenant une population homogène de nanocapsules ayant une taille moyenne inférieure à 500 nanomètres. Le pourcentage de nanocapsules diminue au fur et à mesure qu'augmente la quantité de BSA présente dans la phase aqueuse et est pratiquement nul lorsque cette quantité est égale à 300 mg de BSA par millilitre de phase aqueuse.

## Revendications

1. Composition pharmaceutique sous forme d'une suspension colloïdale de nanocapsules caractérisée en ce qu'elle comprend une phase huileuse constituée essentiellement par une huile contenant en solution un agent tensioactif et en suspension, des nanocapsules d'un diamètre inférieur à 500 nanomètres, renfermant une phase aqueuse constituée essentiellement par une solution ou une suspension d'une substance à activité thérapeutique et d'un agent tensioactif dans l'eau, dont le pH est compris entre 1 et 7, la paroi desdites nanocapsules étant formée par un poly(2-cyanoacrylate d'alkyle) dont le radical alkyle renferme de 1 à 6 atomes de carbone.

2. Composition pharmaceutique selon la revendication 1, caractérisée en ce que la substance à activité thérapeutique est un polypeptide ou un polysaccharide.

3. Composition pharmaceutique selon l'une quelconque des revendications 1 et 2, caractérisée en ce que la substance à activité thérapeutique est la calcitonine, la somatostatine, l'insuline ou l'héparine.

4. Composition pharmaceutique selon l'une quelconque des revendications 1 à 3, caractérisée en ce que la phase aqueuse contient une quantité d'éthanol comprise entre 100 µl et 1.000 µl, de préférence entre 200 µl et 500 µl par millilitre de phase aqueuse.

5. Composition pharmaceutique selon l'une quelconque des revendications 1 à 4, caractérisée en ce que l'huile est une huile végétale, une huile minérale ou un composé huileux choisi parmi le benzoate de benzyle et les glycérides d'acides gras supérieurs.

6. Composition pharmaceutique selon l'une quelconque des revendications 1 à 5, caractérisée en ce que le rapport en volume de la phase aqueuse et de la phase huileuse est compris entre 1/100 et 20/100, de préférence entre 5/100 et 15/100.

7. Composition pharmaceutique selon l'une quelconque des revendications 1 à 6, caractérisée en ce que l'agent tensioactif dans la phase aqueuse est choisi parmi le laurylsulfate de sodium, le dioctylsulfosuccinate de sodium, les esters d'acides gras de sorbitanne polyoxyéthylénés, les dérivés mixtes d'oxyde d'éthylène et de propylène glycol et le polyéthoxyéthylène glycol, de préférence le laurylsulfate de sodium.

8. Composition pharmaceutique selon l'une quelconque des revendications 1 à 7, caractérisée en ce que la concentration de l'agent tensioactif dans la phase aqueuse est comprise entre 0,1% et 10%, de préférence entre 3% et 5% en poids d'agent tensioactif par volume de phase aqueuse.

9. Composition pharmaceutique selon l'une quelconque des revendications 1 à 8, caractérisée en ce que l'agent tensioactif dans la phase huileuse est choisi parmi les esters d'acides gras de sorbitanne et les sels solubles d'acides biliaires, de préférence le mono-oléate de sorbitanne.

10. Composition pharmaceutique selon l'une quelconque des revendications 1 à 9, caractérisée en ce que la concentration de l'agent tensioactif dans la phase huileuse est comprise entre 0,1% et 20%, de préférence entre 5% et 15% en poids d'agent tensioactif par volume de phase huileuse.

11. Procédé de préparation d'une composition pharmaceutique selon l'une quelconque des revendications 1 à 10, caractérisé en ce que :
(a) on prépare une phase aqueuse constituée essentiellement par une solution ou une suspension d'une substance à activité thérapeutique et d'un agent tensioactif dans l'eau, dont le pH est compris entre 1 et 7 ;
(b) on ajoute lentement, sous agitation, cette phase aqueuse dans une phase huileuse constituée essentiellement par une huile contenant en solution un agent tensioactif ;
(c) on ajoute, sous agitation, à l'émulsion de type eau dans l'huile ainsi obtenue, au moins un 2-cyanoacrylate d'alkyle dont le radical alkyle renferme de 1 à 6 atomes de carbone, tel quel, sans solvant, puis on laisse la polymérisation s'effectuer à la température ambiante pendant une durée de temps suffisante pour que le 2-cyanoacrylate d'alkyle introduit soit polymérisé, et on recueille comme produit du procédé une suspension dans la phase huileuse de nanocapsules de diamètre inférieur à 500 nanomètres, renfermant ladite phase aqueuse.

12. Procédé selon la revendication 11, caractérisé en ce que la polymérisation du 2-cyanoacylate d'alkyle est effectuée pendant une durée de 3 à 8 heures.

13. Procédé selon l'une quelconque des revendications 11 et 12, caractérisé en ce que la substance à activité thérapeutique est la calcitonine, la somatostatine, l'insuline ou l'héparine.

14. Procédé selon l'une quelconque des revendications 11 à 13, caractérisé en ce que la phase aqueuse contient une quantité d'éthanol comprise entre 100 µl et 1.000 µl, de préférence entre 200 µl et 500 µl par millilitre de phase aqueuse.

15. Procédé selon l'une quelconque des revendications 11 à 14, caractérisé en ce que l'agent tensioactif dans la phase aqueuse est le laurylsulfate de sodium.

16. Procédé selon l'une quelconque des revendications 11 à 15, caractérisé en ce que la concentration de l'agent tensioactif dans la phase aqueuse est compris entre 0,1% et 10%, de préférence entre 3% et 5% en poids d'agent tensioactif par volume de phase aqueuse.

17. Procédé selon l'une quelconque des revendications 11 à 16, caractérisé en ce que l'agent tensioactif dans la phase huileuse est le mono-oléate de sorbitanne.

18. Procédé selon l'une quelconque des revendications 11 à 17, caractérisé en ce que la concentration de l'agent tensioactif dans la phase huileuse est comprise entre 0,1% et 20%, de préférence entre 5% et 15% en poids d'agent tensioactif par volume de phase huileuse.

## Claims

1. A pharmaceutical composition in the form of a colloidal suspension of nanocapsules, characterised in that it comprises an oily phase consisting essentially of an oil containing dissolved therein a surfactant and, suspended therein, nanocapsules having a diameter of less than 500 nanometers, said nanocapsules encapsulating an aqueous phase consisting essentially of a solution or a suspension of a therapeutically active substance and a surfactant in water, whose pH lies between 1 and 7, the walls of said nanocapsules being formed from a poly(alkyl-2-cyanoacrylate) wherein the alkyl radical has 1 to 6 carbon atoms.

2. A pharmaceutical composition according to claim 1, characterised in that the therapeutically active substance is a polypeptide or a polysaccharide.

3. A pharmaceutical composition according to anyone of claims 1 and 2, characterised in that the therapeutically active substance is calcitonin, somatostatin, insulin or heparin.

4. A pharmaceutical composition according to anyone of claims 1 to 3, characterised in that the aqueous phase contains a quantity of ethanol comprised between 100 µl and 1,000 µl, preferably between 200 µl and 500 µl per millilitre of aqueous phase.

5. A pharmaceutical composition according to anyone of claims 1 to 4, characterised in that the oil is a vegetable oil, a mineral oil or an oily compound selected from benzyl benzoate and glycerides of higher fatty acids.

6. A pharmaceutical composition according to anyone of claims 1 to 5, characterised in that the ratio by volume of the aqueous phase to the oily phase is comprised between 1/100 and 20/100, preferably between 5/100 and 15/100.

7. A pharmaceutical composition according to anyone of claims 1 to 6, characterised in that the surfactant in the aqueous phase is selected from sodium laurylsulfate, sodium dioctylsulfosuccinate, polyoxyethylene sorbitan fatty acid esters, mixed derivatives of ethylene oxide and propylene glycol and polyoxyethylene glycol, preferably sodium laurylsulfate.

8. A pharmaceutical composition according to anyone of claims 1 to 7, characterised in that the concentration of surfactant in the aqueous phase is comprised between 0.1% and 10%, preferably between 3% and 5% by weight of surfactant per volume of aqueous phase.

9. A pharmaceutical composition according to anyone of claims 1 to 8, characterised in that the surfactant in the oily phase is selected from sorbitan fatty acid esters and soluble salts of bile acids, preferably sorbitan mono-oleate.

10. A pharmaceutical composition according to anyone of claims 1 to 9, characterised in that the concentration of surfactant in the oily phase is between 0.1% and 20%, preferably between 5% and 15% by height of the surfactant per volume of oily phase.

11. A process for the preparation of a pharmaceutical composition according to anyone of claims 1 to 10, characterised in that:
a) an aqueous phase is prepared consisting essentially of a solution or a suspension of a therapeutically active substance and a surfactant in water, whose pH lies between 1 and 7;
b) said aqueous phase is added slowly under stirring into an oily phase consisting essentially of an oil containing a surfactant dissolved therein;
c) at least one alkyl 2-cyanoacrylate, wherein the alkyl radical has 1 to 6 carbon atoms, is added under stirring to the water-in-oil type emulsion so obtained, just as it is, in the absence of a solvent, then the polymerisation is allowed to take place at ambient temperature for a sufficient period of time to polymerize the added alkyl 2-cyanoacrylate, and a suspension in the oily phase of nanocapsules having a diameter of less than 500 nanometers, encapsulating said aqueous phase, is recovered as product of the process.

12. A process according to claim 11, characterised in that polymerization of the alkyl 2-cyanoacrylate is carried out for a period of 3 to 8 hours.

13. A process according to anyone of claims 11 or 12, characterised in that the therapeutically active substance is calcitonin, somatostatin insulin or heparin.

14. A process according to anyone of claims 11 to 13, characterised in that the aqueous phase contains a quantity of ethanol comprised between 100 µl and 1,000 µl, preferably between 200 µl and 500 µl per millilitre thereof.

15. A process according to anyone of claims 11 to 14, characterised in that the surfactant in the aqueous phase is sodium laurylsulfate.

16. A process according to anyone of claims 11 to 15, characterised in that the concentration of surfactant in the aqueous phase is comprised between 0.1% and 10%, preferably between 3% and 5% by weight of surfactant per volume of aqueous phase.

17. A process according to anyone of claims 11 to 16, characterised in that the surfactant in the oily phase is sorbitan mono-oleate.

18. A process according to anyone of claims 11 to 17, characterised in that the concentration of surfactant in the oily phase is comprised between 0.1% and 20%, preferably between 5% and 15% by weight of the surfactant per volume of oily phase.

## Patentansprüche

1. Pharmazeutische Zusammensetzung in Form einer kolloidalen Suspension von Nanokapseln, dadurch gekennzeichnet, daß sie eine ölige Phase umfaßt, die im wesentlichen aus einem Öl besteht, das in Lösung ein Tensid und in Suspension Nanokapseln mit einem Durchmesser kleiner als 500 Nanometer enthält, die eine wäßrige Phase einschließen, die im wesentlichen aus einer Lösung oder einer Suspension einer Substanz mit therapeutischer Wirksamkeit und eines Tensids in Wasser besteht, deren pH-Wert zwischen 1 und 7 liegt, wobei die Wand besagter Nanokapseln durch ein Poly(alkyl-2-cyanoacrylat), dessen Alkylrest 1 bis 6 Kohlenstoffatome enthält, gebildet wird.

2. Pharmazeutische Zusammensetzung gemäß Anspruch 1, dadurch gekennzeichnet, daß die Substanz mit therapeutischer Wirksamkeit ein Polypeptid oder ein Polysaccharid ist.

3. Pharmazeutische Zusammensetzung gemaß einem der Ansprüche 1 und 2, dadurch gekennzeichnet, daß die Substanz mit therapeutischer Wirksamkeit Calcitonin, Somatostatin, Insulin oder Heparin ist.

4. Pharmazeutische Zusammensetzung gemäß einem der Ansprüche 1 bis 3, dadurch gekennzeichnet, daß die wäßrige Phase eine Menge an Ethanol enthält, die zwischen 100 µl und 1.000 µl, vorzugsweise zwischen 200 µl und 500 µl pro Milliliter wäßriger Phase liegt.

5. Pharmazeutische Zusammensetzung gemäß einem der Ansprüche 1 bis 4, dadurch gekennzeichnet, daß das Öl ein Pflanzenöl, ein Mineralöl oder eine ölige Verbindung, ausgewählt unter Benzylbenzoat und den Glyceriden höherer Fettsäuren, ist.

6. Pharmazeutische Zusammensetzung gemäß einem der Ansprüche 1 bis 5, dadurch gekennzeichnet, daß das Volumenverhältnis der wäßrigen Phase und der öligen Phase zwischen 1/100 und 20/100, vorzugsweise zwischen 5/100 und 15/100 liegt.

7. Pharmazeutische Zusammensetzung gemäß einem der Ansprüche 1 bis 6, dadurch gekennzeichnet. daß das Tensid in der wäßrigen Phase unter Natriumlaurylsulfat, Natriumdioctylsulfosuccinat, den Polyoxyethylen-Sorbitanfettsäureestem, den Mischderivaten von Ethylenoxid und Propylenglykol und dem Polyethoxyethylenglykol, vorzugsweise dem Natriumlaurylsulfat ausgewählt ist.

8. Pharmazeutische Zusammensetzung gemäß einem der Ansprüche 1 bis 7. dadurch gekennzeichnet, daß die Konzentration des Tensids in der wäßrigen Phase zwischen 0,1 Gew.-% und 10 Gew.-%, vorzugsweise zwischen 3 Gew.-% und 5 Gew.-% Tensid pro Volumen wäßriger Phase liegt.

9. Pharmazeutische Zusammensetzung gemäß einem der Ansprüche 1 bis 8, dadurch gekennzeichnet, daß das Tensid in der öligen Phase unter den Sorbitanfettsäureestern und den löslichen Salzen von Gallensäuren, vorzugsweise dem Sorbitanmonooleat ausgewählt ist.

10. Pharmazeutische Zusammensetzung gemäß einem der Ansprüche 1 bis 9, dadurch gekennzeichnet; daß die Konzentration des Tensids in der öligen Phase zwischen 0,1 Gew.-% und 20 Gew.-%, vorzugsweise zwischen 5 Gew.-% und 15 Gew.-% Tensid pro Volumen öliger Phase liegt.

11. Verfahren zur Herstellung einer pharmazeutischen Zusammensetzung gemäß einem der Ansprüche 1 bis 10, dadurch gekennzeichnet, daß:
(a) man eine wäßrige Phase herstellt, die im wesentlichen aus einer Lösung oder einer Suspension einer Substanz mit therapeutischer Wirksamkeit und eines Tensids in Wasser besteht, deren pH-Wert zwischen 1 und 7 liegt;
(b) man diese wäßrige Phase unter Rühren langsam in eine ölige Phase gibt, die im wesentlichen aus einem Öl besteht, das ein Tensid in Lösung enthält;
(c) man unter Rühren zu der so erhaltenen Emulsion vom Wasser-in-Öl-Typ wenigstens ein Alkyl-2-cyanoacrylat, dessen Alkylrest 1 bis 6 Kohlenstoffatome enthält, so, wie es ist, ohne Lösungsmittel hinzufügt, man dann sich die Polymerisation bei Raumtemperatur während einer Zeitdauer, die ausreicht, damit das eingebrachte Alkyl-2-cyanoacrylat polymerisiert wird, erfolgen läßt, und man als Produkt des Verfahrens eine Suspension von Nanokapseln mit einem Durchmesser kleiner als 500 Nanometer, die besagte wäßrige Phase einschließen, in der öligen Phase gewinnt.

12. Verfahren gemäß Anspruch 11, dadurch gekennzeichnet. daß die Polymerisation des Alkyl-2-cyanoacrylats während einer Dauer von 3 bis 8 Stunden ausgeführt wird.

13. Verfahren gemäß einem der Ansprüche 11 und 12, dadurch gekennzeichnet, daß die Substanz mit therapeutischer Wirksamkeit Calcitonin Somatostatin, Insulin oder Heparin ist.

14. Verfahren gemäß einem der Ansprüche 11 bis 13, dadurch gekennzeichnet, daß die wäßrige Phase eine Menge an Ethanol enthält, die zwischen 100 µl und 1.000 µl, vorzugsweise zwischen 200 µl und 500 µl pro Milliliter wäßriger Phase liegt.

15. Verfahren gemäß einem der Ansprüche 11 bis 14, dadurch gekennzeichnet, daß das Tensid in der wäßrigen Phase Natriumlaurylsulfat ist.

16. Verfahren gemäß einem der Ansprüche 11 bis 15, dadurch gekennzeichnet, daß die Konzentration des Tensids in der wäßrigen Phase zwischen 0,1 Gew.-% und 10 Gew.-%, vorzugsweise zwischen 3 Gew.-% und 5 Gew.-% Tensid pro Volumen wäßriger Phase liegt.

17. Verfahren gemäß einem der Ansprüche 11 bis 16, dadurch gekennzeichnet, daß das Tensid in der öligen Phase Sorbitanmonooleat ist.

18. Verfahren gemäß einem der Ansprüche 11 bis 17, dadurch gekennzeichnet, daß die Konzentration des Tensids in der öligen Phase zwischen 0,1 Gew.-% und 20 Gew.-%, vorzugsweise zwischen 5 Gew.-% und 15 Gew.-% Tensid pro Volumen öliger Phase liegt.
